# EUROPEAN PATENT APPLICATION

(11) **EP 4 393 917 A1**
(43) Date of publication of application: **03.07.2024**
(21) Application number: 22869392.5
(22) Date of filing: 16.09.2022
(51) Int. Cl.: C07D 403/14, A61K 31/506, A61P 9/00, A61P 25/00, A61P 35/00, A61P 11/00

(54) **SOLID FORM OF RHO-ASSOCIATED PROTEIN KINASE INHIBITOR OR SOLVATE THEREOF, PREPARATION METHOD AND USE THEREOF**

(30) Priority: 18.09.2021 CN 202111103170
(71) Applicant: Beijing Tide Pharmaceutical Co., Ltd., Beijing 100176 (CN)
(72) Inventor: WANG, Hongjun, Beijing 100176 (CN); FENG, Zewang, Beijing 100176 (CN); TIAN, Nana, Beijing 100176 (CN); ZHAO, Yanping, Beijing 100176 (CN); YANG, Jun, Beijing 100176 (CN); WEI, Lai, Beijing 100176 (CN); CAO, Xiangrong, Beijing 100176 (CN); CHEN, Jie, Beijing 100176 (CN)
(74) Representative: Turner, Craig Robert
(86) International application number: PCT/CN2022/119269
(87) International publication number: WO 2023/041026

(57) **Abstract**

Provided are a solid form of a Rho-associated protein kinase inhibitor (6-(4-((4-(1H-pyrazol-4-yl)phenyl)amino)pyrimidin-2-yl)-1-methyl-1H-indol-2-yl)(3,3-difluoroazetidin-1-yl)methanone or a solvate thereof, a method for preparing the solid form, a pharmaceutical composition comprising the solid form, and a use of the solid form as a Rho-associated protein kinase (ROCK) inhibitor, preferably a selective ROCK2 inhibitor.

## Description

### FIELD OF THE INVENTION

The present invention relates to a solid form of a Rho-associated protein kinase inhibitor (6-(4-((4-(1H-pyrazol-4-yl)phenyl)amino)pyrimidin-2-yl)-1-methyl-1H-indol-2-yl)(3,3-difluoroazetidin-1-yl)methanone (hereinafter referred to as "Compound A") or a solvate thereof, a method for preparing the solid form, a pharmaceutical composition comprising the solid form, and a use of the solid form as a Rho-associated protein kinase (ROCK) inhibitor, preferably a selective ROCK2 inhibitor.

### BACKGROUND OF THE INVENTION

Rho-associated protein kinase (ROCK) is a serine/threonine kinase from the AGC kinase family, and comprises two isoforms, ROCK1 and ROCK2. ROCK1 and ROCK2 are expressed and regulated differently in specific tissues. For example, ROCK1 is ubiquitously expressed at a relatively high level, while ROCK2 is preferentially expressed in heart, brain and skeletal muscle. ROCK is the first downstream effector of the Rho protein discovered, and its biological function is achieved by phosphorylating the downstream effector proteins (MLC, Lin-11, Isl-1, LIMK, ERM, MARCKS, CRMP-2, *etc*.). Studies have shown that various diseases (e.g., pulmonary fibrosis, cardiac-cerebral vascular disease, neurological disease and cancer *etc*.) are related to the pathways mediated by ROCK. As such, ROCK is considered as an important target in the development of novel drugs.

The applicant has discovered that (6-(4-((4-(1H-pyrazol-4-yl)phenyl)amino)pyrimidin-2-yl)-1-methyl-1H-indol-2-yl)(3,3-difluoroazetidin-1-yl)methanone can be used as an effective Rho-associated protein kinase (ROCK) inhibitor (see PCT/CN2018/093713, which is incorporated herein in its entirety by reference), but there have been no reports on the solid forms of this compound.

### SUMMARY OF THE INVENTION

In one aspect, the present invention provides crystalline forms of compound A ((6-(4-((4-(1H-pyrazol-4-yl)phenyl)atnino)pyrimidin-2-yl)-1-methyl-1H-indol-2-yl)(3,3-difluoroazetidin-1-yl)methanone) as shown below or a solvate thereof:

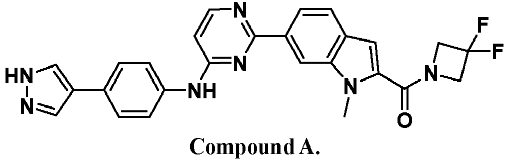

The preferred crystalline forms of the present invention not only have an excellent effect in preventing or treating a disease mediated by the Rho-associated protein kinase (ROCK), but also have additional advantages. For example, the preferred crystalline forms of the present invention have excellent physical properties (including solubility, dissolution rate, light resistance, low hygroscopicity, high temperature resistance, high humidity resistance, fluidity, and the like), and the preferred crystalline forms of the present invention may have superior properties in terms of bioavailability, physical and/or chemical stability, and ease of preparation. The preferred crystalline forms of the present invention have good powder properties, are more suitable and convenient for mass production and for forming a formulation, can reduce irritation and enhance absorption, solve problems in metabolic rates, significantly decrease toxicity resulted from drug accumulation, improve safety, and effectively ensure the quality and efficacy of the pharmaceutical products.

In another aspect, the present invention provides methods for preparing the crystalline forms of the present invention.

In another aspect, the present invention provides a pharmaceutical composition comprising any one or more of the crystalline forms of the present invention and one or more pharmaceutically acceptable carriers.

In another aspect, the present invention provides use of the crystalline form of the present invention in the manufacture of a medicament as a Rho-associated protein kinase (ROCK) inhibitor, preferably a selective ROCK2 inhibitor.

### BRIEF DESCRIPTION OF THE DRAWINGS

Figure 1 is the X-ray powder diffraction pattern of Crystalline Form I of Compound A monohydrate.
Figure 2 is the differential scanning calorimetry (DSC) graph of Crystalline Form I of Compound A monohydrate.
Figure 3 is the thermogravimetric analysis (TGA) graph of Crystalline Form I of Compound A monohydrate.
Figure 4 is the scanning electron microscope image of Crystalline Form I of Compound A monohydrate.
Figure 5 is the X-ray powder diffraction pattern of Crystalline Form II of Compound A anhydrate.
Figure 6 is the differential scanning calorimetry (DSC) graph of Crystalline Form II of Compound A anhydrate.
Figure 7 is the thermogravimetric analysis (TGA) graph of Crystalline Form II of Compound A anhydrate.
Figure 8 is the scanning electron microscope image of Crystalline Form II of Compound A anhydrate.
Figure 9 is the X-ray powder diffraction pattern of Crystalline Form III of Compound A monohydrate.
Figure 10 is the differential scanning calorimetry (DSC) graph of Crystalline Form III of Compound A monohydrate.
Figure 11 is the thermogravimetric analysis (TGA) graph of Crystalline Form III of Compound A monohydrate.
Figure 12 is the scanning electron microscope image of Crystalline Form III of Compound A monohydrate.
Figure 13 is the X-ray powder diffraction pattern of Crystalline Form IV of Compound A monohydrate.
Figure 14 is the differential scanning calorimetry (DSC) graph of Crystalline Form IV of Compound A monohydrate.
Figure 15 is the thermogravimetric analysis (TGA) graph of Crystalline Form IV of Compound A monohydrate.
Figure 16 is the scanning electron microscope image of Crystalline Form IV of Compound A monohydrate.
Figure 17 is the X-ray powder diffraction pattern of Crystalline Form V of Compound A monohydrate.
Figure 18 is the differential scanning calorimetry (DSC) graph of Crystalline Form V of Compound A monohydrate.
Figure 19 is the thermogravimetric analysis (TGA) graph of Crystalline Form V of Compound A monohydrate.
Figure 20 is the comparative X-ray powder diffraction pattern of Crystalline Form II of Compound A anhydrate in the solid stability test.
Figure 21 is the comparative X-ray powder diffraction pattern of Crystalline Form I of Compound A monohydrate before and after heat treatment.

### DETAILED DESCRIPTION OF THE INVENTION

### DEFINITIONS

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that most of the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

The word "about" as used herein refers to, as appreciated by a person skilled in the art, a range within the acceptable standard error of a value, such as ±0.05, ±0.1, ±0.2, ±0.3, ±1, ±2 or ±3, *etc.*

The term "solid form" as used herein includes all solid forms of compound A or any solvate thereof, such as a crystalline form or amorphous form.

The term "amorphous" as used herein refers to any solid substance which lacks order in three dimensions. In some instances, amorphous solids may be characterized by known techniques, including XRPD crystallography, solid state nuclear magnet resonance (ssNMR) spectroscopy, DSC, or some combination of these techniques. As illustrated below, amorphous solids give diffuse XRPD patterns, typically comprised of one or two broad peaks (i.e., peaks having base widths of about 5° 2Θ or greater).

The term "crystalline form" or "crystal" as used herein refers to any solid substance exhibiting three-dimensional order, which in contrast to an amorphous solid substance, gives a distinctive XRPD pattern with sharply defined peaks.

The term "X-ray powder diffraction pattern (XRPD pattern)" as used herein refers to the experimentally observed diffractogram or parameters derived therefrom. XRPD patterns are usually characterized by peak positions (abscissa) and peak intensities (ordinate).

The term "20" as used herein refers to the peak position in degrees based on the experimental setup of the X-ray diffraction experiment and is a common abscissa unit in diffraction patterns. The experimental setup requires that if a reflection is diffracted when the incoming beam forms an angle theta (θ) with a certain lattice plane, the reflected beam is recorded at an angle 2 theta (2θ). It should be understood that reference herein to specific 2θ values for a specific solid form is intended to mean the 2θ values (in degrees) as measured using the X-ray diffraction experimental conditions as described herein. For example, as described herein, Cu-Kα (Kα1 (Å): 1.540598 and Kα2 (Å): 1.544426 Å) was used as the source of radiation.

As used herein, "I%" refers to the percentage of peak intensity.

The term "differential scanning calorimetry (DSC) graph" as used herein refers to a curve recorded on a differential scanning calorimeter.

The term "thermogravimetric analysis (TGA) graph" as used herein refers to a curve recorded on a thermogravimetric analyzer.

As used herein, the term "essentially the same" with reference to X-ray diffraction peak positions means that typical peak position and intensity variability are taken into account. For example, one skilled in the art will appreciate that the peak positions (2Θ) will show some variability, typically as much as 0.1 to 0.2 degree, as well as on the apparatus being used to measure the diffraction. Further, one skilled in the art will appreciate that relative peak intensities will show inter-apparatus variability as well as variability due to degree of crystallinity, preferred orientation, prepared sample surface, and other factors known to those skilled in the art. Similarly, as used herein, "essentially the same" with reference to the DSC graph is intended to also encompass the variabilities associated with these analytical techniques, which are known to those of skill in the art. For example, a differential scanning calorimetry graph will typically have a variability of up to ±0.2°C for well defined peaks, and even larger for broad lines (e.g., up to ±1°C).

The liquid nuclear magnetic resonance spectrum in the present application is preferably collected on a Bruker 400M nuclear magnetic resonance spectrometer, with DMSO-*d₆* as the solvent, unless otherwise stated.

The polarization microscopy data in the present application is preferably collected on Polarizing Microscope ECLIPSE LV100POL (Nikon, JPN).

Numerical ranges (e.g., "1 to 10", "1 to 6", "2 to 10", "2 to 6", "3 to 10", "5 to 10", "3 to 6"), *etc.* as used herein encompass any point within the numerical range (for example, 1, 2, 3, 4, 5, 6, 7, 8, 9, or 10).

The prepared crystalline form may be recovered by methods including decantation, centrifugation, evaporation, gravity filtration, suction filtration, or any other technique for the recovery of solids under pressure or under reduced pressure. The recovered solid may optionally be dried. "Drying" in the present invention is carried out under reduced pressure (preferably in vacuum) until the residual solvent content is lowered within the limits given in the International Conference on Harmonisation of Technical Requirements for Registration of Pharmaceuticals for Human Use ("ICH") guidelines. The residual solvent content depends on the type of the solvent, but does not exceed about 5000 ppm, or preferably about 4000 ppm, or more preferably about 3000 ppm. Drying may be carried out in a tray dryer, vacuum oven, air oven, cone vacuum dryer, rotary vacuum dryer, fluidized bed dryer, spin flash dryer, flash dryer, or the like. The drying may be carried out at temperatures less than about 100°C, less than about 80°C, less than about 60°C, less than about 50°C, less than about 30°C, or any other suitable temperatures, at atmospheric pressure or under a reduced pressure (preferably in vacuum) for any desired period (e.g., about 1, 2, 3, 5, 10, 15, 20, 24 hours or overnight) until the desired result is achieved, as long as the crystalline form is not degraded in quality. The drying can be carried out any desired times until the desired product quality is achieved. The dried product may optionally be subjected to a size reduction procedure to produce desired particle sizes. Milling or micronization may be performed before drying, or after the completion of drying of the product. Techniques that may be used for particle size reduction include, without limitation, ball, roller and hammer milling, and jet milling.

The term "anhydrate" as used herein preferably means a crystalline form wherein no water molecule is comprised as a structural element.

### Crystalline form of Compound A and preparation method therefor

In an embodiment, the present invention provides Crystalline Form I of Compound A monohydrate: 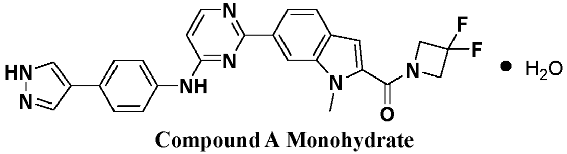 wherein the Crystalline Form I has an XRPD pattern comprising characteristic peaks at diffraction angles (2θ) of about 4.5±0.2°, 12.2±0.2°, 20.1±0.2°, 25.3±0.2° and 25.5±0.2°.

In a preferred embodiment, the Crystalline Form I has an XRPD pattern comprising characteristic peaks at diffraction angles (2θ) of about 4.5±0.2°, 9.1±0.2°, 10.3±0.2°, 12.2±0.2°, 16.1±0.2°, 18.2±0.2°, 20.1±0.2°, 25.3±0.2° and 25.5±0.2°.

In a more preferred embodiment, the Crystalline Form I has an XRPD pattern comprising characteristic peaks at diffraction angles (2θ) of about 4.5±0.2°, 9.1±0.2°, 10.3±0.2°, 12.2±0.2°, 12.5±0.2°, 16.1±0.2°, 16.9±0.2°, 17.4±0.2°, 18.2±0.2°, 18.9±0.2°, 20.1±0.2°, 20.5±0.2°, 21.2±0.2°, 22.6±0.2°, 24.3±0.2°, 24.8±0.2°, 25.3±0.2°, 25.5±0.2°, 26.8±0.2° and 27.2±0.2°.

In a more preferred embodiment, the Crystalline Form I has an XRPD pattern comprising peaks at the following diffraction angles (2θ):

| **Peak No.** | **2θ (°) ± 0.2°** | **I%** | **Peak No.** | **2θ (°) ± 0.2°** | **I%** | **Peak No.** | **2θ (°) ± 0.2°** | **I%** |
|---|---|---|---|---|---|---|---|---|
| 1 | 4.5 | 62.3 | 15 | 16.1 | 28.8 | 29 | 24.3 | 20 |
| 2 | 7.7 | 7.1 | 16 | 16.9 | 11.4 | 30 | 24.8 | 22.4 |
| 3 | 8.5 | 9.5 | 17 | 17.4 | 14.4 | 31 | 25.3 | 76.6 |
| 4 | 9.1 | 33.5 | 18 | 18.2 | 29.2 | 32 | 25.5 | 100 |
| 5 | 10.3 | 26.9 | 19 | 18.9 | 12.3 | 33 | 26.8 | 16.9 |
| 6 | 10.8 | 9.4 | 20 | 20.1 | 37.8 | 34 | 27.2 | 13 |
| 7 | 11.2 | 6.3 | 21 | 20.5 | 12.4 | 35 | 28.2 | 9.9 |
| 8 | 12.2 | 46.8 | 22 | 21.2 | 11.6 | 36 | 28.8 | 6.5 |
| 9 | 12.5 | 15.9 | 23 | 21.8 | 6.6 | 37 | 31.7 | 5.5 |
| 10 | 13.1 | 9.7 | 24 | 22.3 | 9.8 | 38 | 33.3 | 4.2 |
| 11 | 13.4 | 8.5 | 25 | 22.6 | 10.3 | 39 | 36.3 | 3.9 |
| 12 | 13.8 | 7 | 26 | 23.5 | 9.7 | 40 | 36.5 | 3.5 |
| 13 | 14.3 | 7.3 | 27 | 23.6 | 9.1 | 41 | 38.0 | 3.4 |
| 14 | 15.3 | 8.9 | 28 | 23.7 | 9.2 | 42 | 38.3 | 3.5 |

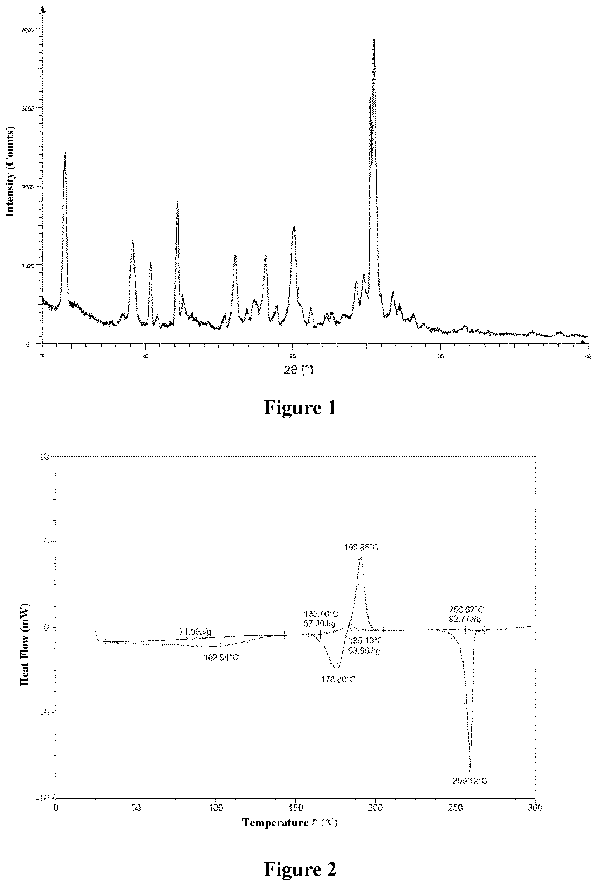

In a more preferred embodiment, the Crystalline Form I has an XRPD pattern comprising peaks at diffraction angles (2Θ) essentially the same as shown in Figure 1. In the most preferred embodiment, the XRPD pattern of the Crystalline Form I is essentially the same as shown in Figure 1.

In a more preferred embodiment, the Crystalline Form I has a DSC graph comprising a broad endothermic peak at about 103°C, a broad endothermic peak at about 177°C, an exothermic peak at about 191°C, and an endothermic peak at about 259°C.

In a more preferred embodiment, the Crystalline Form I has a DSC graph comprising characteristic peaks essentially the same as shown in Figure 2. In the most preferred embodiment, the DSC graph of the Crystalline Form I is essentially the same as shown in Figure 2.

In a more preferred embodiment, in a thermogravimetric analysis, the Crystalline Form I has a weight loss of about 1.5% when heated to about 68°C, a weight loss of about 2.2% when heated to about 68-147°C, and a weight loss of about 0.3% when heated to about 147-200°C.

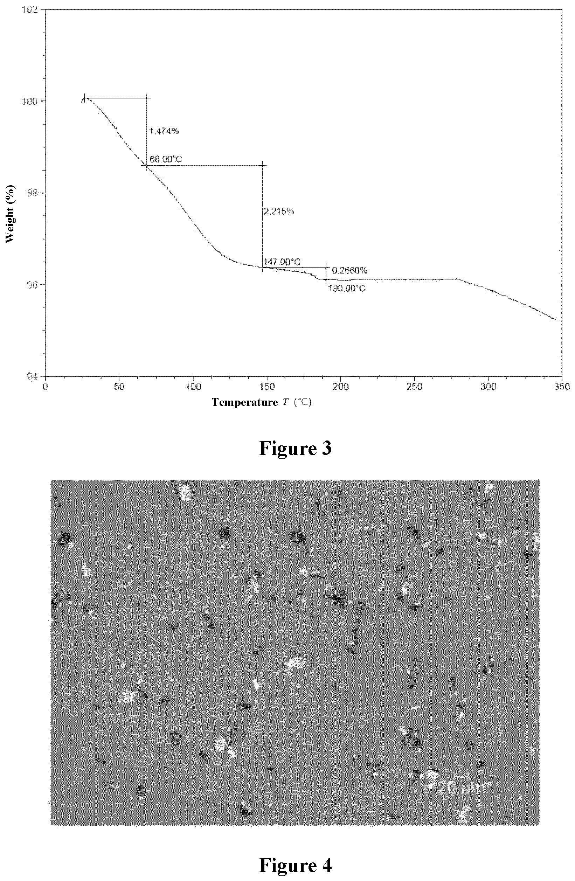

In the most preferred embodiment, the TGA graph of the Crystalline Form I is essentially the same as shown in Figure 3.

In the most preferred embodiment, the scanning electron microscope image of the Crystalline Form I is essentially the same as shown in Figure 4.

In some embodiments, the present invention provides a method for preparing the Crystalline Form I, which comprises stirring Compound A in a mixed solvent comprising a hydrocarbon with 1-10 carbon atoms, an amide with 1-10 carbon atoms, and water (for example, at room temperature, for about 1-30 hours, preferably about 10-20 hours), filtering, rinsing the filter cake with water, and vacuum drying to afford the crystal.

In a preferred embodiment, the weight-to-volume ratio (g/mL) of Compound A to the mixed solvent is about 1:(1-10), preferably about 1:3.5.

In a preferred embodiment, the hydrocarbon solvent with 1-10 carbon atoms includes alkanes, halogenated alkanes, alkenes, alkynes, and aromatic hydrocarbons, specifically including but not limited to dichloromethane, trichloromethane (chloroform), n-hexane, n-heptane, and toluene.

In a preferred embodiment, the amide solvent with 1-10 carbon atoms is N,N-dimethylformamide or N,N-dimethylacetamide.

In a preferred embodiment, the volume ratio of the hydrocarbon with 1-10 carbon atoms, amide with 1-10 carbon atoms, and water in the mixed solvent is about 5:1:1.

In a preferred embodiment, the vacuum drying is carried out at about 20-50°C (preferably about 40°C), for about 1-10 hours (preferably 6 hours).

In an embodiment, the present invention provides Crystalline Form II of Compound A anhydrate: 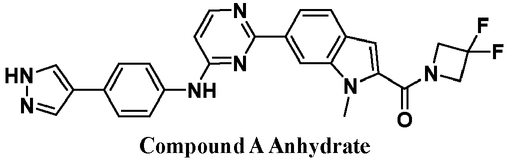 wherein the Crystalline Form II has an XRPD pattern comprising characteristic peaks at diffraction angles (2Θ) of about 8.6±0.2°, 12.5±0.2° and 21.9±0.2°.

In a preferred embodiment, the Crystalline Form II has an XRPD pattern comprising characteristic peaks at diffraction angles (2θ) of about 6.7±0.2°, 8.6±0.2°, 12.5±0.2°, 13.5±0.2°, 13.8±0.2°, 21.9±0.2° and 24.9±0.2°.

In a more preferred embodiment, the Crystalline Form II has an XRPD pattern comprising characteristic peaks at diffraction angles (2θ) of about 6.2±0.2°, 6.7±0.2°, 8.6±0.2°, 11.1±02°, 12.5±0.2°, 13.5±0.2°, 13.8±0.2°, 15.2±0.2°, 17.3±0.2°, 18.2±0.2°, 18.6±0.2°, 21.0±0.2°, 21.9±0.2°, 24.3±0.2°, 24.9±0.2° and 25.9±0.2°.

In a more preferred embodiment, the Crystalline Form II has an XRPD pattern comprising peaks at the following diffraction angles (2θ):

| **Peak No.** | **2θ (°)± 0.2°** | **I%** | **Peak No.** | **2θ (°)± 0.2°** | **I%** | **Peak No.** | **2θ (°)± 0.2°** | **I%** |
|---|---|---|---|---|---|---|---|---|
| 1 | 6.2 | 17.1 | 12 | 18.2 | 10.8 | 23 | 25.9 | 10.8 |
| 2 | 6.7 | 31.7 | 13 | 18.6 | 11.3 | 24 | 27.2 | 8.6 |
| 3 | 8.6 | 44.4 | 14 | 19.0 | 5.1 | 25 | 28.6 | 3.6 |
| 4 | 11.1 | 15.2 | 15 | 19.7 | 8.9 | 26 | 29.0 | 6.4 |
| 5 | 12.5 | 100 | 16 | 20.3 | 6.7 | 27 | 29.7 | 4.6 |
| 6 | 13.5 | 30.5 | 17 | 21.0 | 12.5 | 28 | 30.5 | 5.4 |
| 7 | 13.8 | 29 | 18 | 21.9 | 38 | 29 | 30.7 | 4.8 |
| 8 | 15.2 | 19.6 | 19 | 22.8 | 5 | 30 | 31.7 | 3.4 |
| 9 | 16.3 | 6 | 20 | 23.6 | 6.4 | 31 | 32.8 | 2.4 |
| 10 | 16.8 | 5.2 | 21 | 24.3 | 10.3 | 32 | 33.8 | 2.5 |
| 11 | 17.3 | 20.2 | 22 | 24.9 | 28.3 | | | |

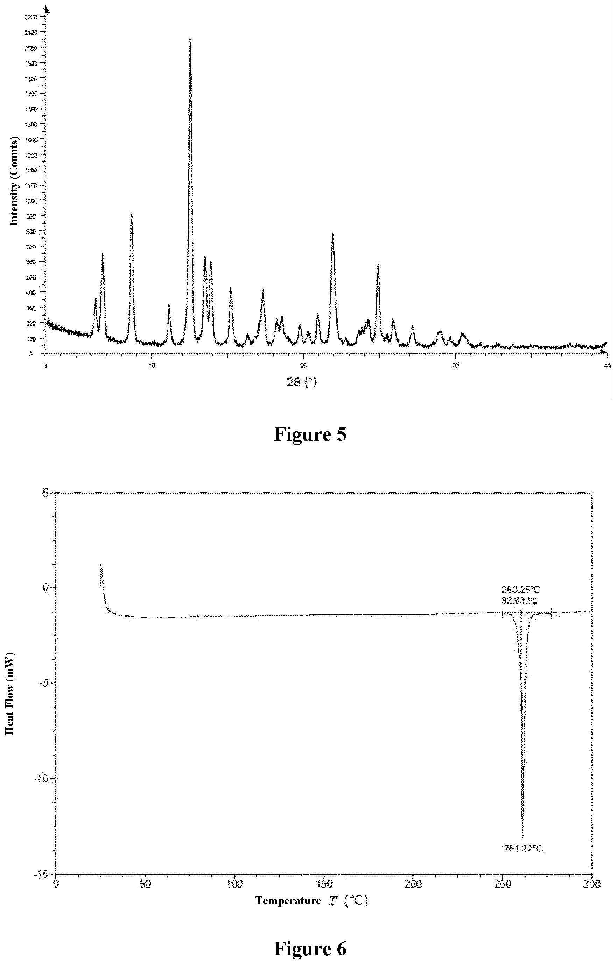

In a more preferred embodiment, the Crystalline Form II has an XRPD pattern comprising peaks at diffraction angles (2θ) essentially the same as shown in Figure 5. In the most preferred embodiment, the XRPD pattern of the Crystalline Form II is essentially the same as shown in Figure 5.

In a more preferred embodiment, the Crystalline Form II has a DSC graph comprising an endothermic peak at about 261°C.

In a more preferred embodiment, the Crystalline Form II has a DSC graph comprising characteristic peaks essentially the same as shown in Figure 6. In the most preferred embodiment, the DSC graph of the Crystalline Form II is essentially the same as shown in Figure 6.

In a more preferred embodiment, in a thermogravimetric analysis, the Crystalline Form II has a weight loss of no more than about 1.0%, preferably no more than about 0.2%, when heated to about 230°C.

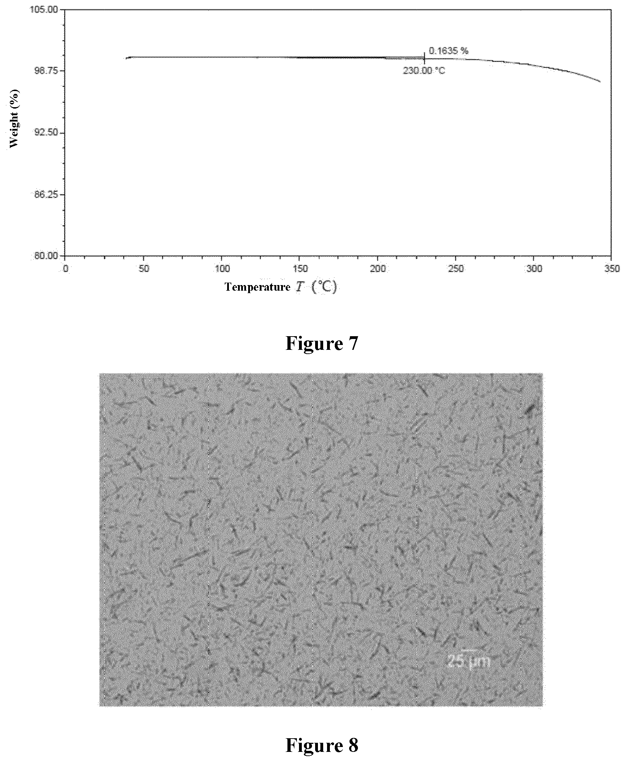

In the most preferred embodiment, the TGA graph of the Crystalline Form II is essentially the same as shown in Figure 7.

In the most preferred embodiment, the scanning electron microscope image of the Crystalline Form II is essentially the same as shown in Figure 8.

In some embodiments, the present invention provides a method for preparing the Crystalline Form II, which comprises stirring Compound A in an ester solvent with 2-10 carbon atoms (for example, at about 20-80°C (for example, about 55°C), for about 1-30 hours, preferably about 10-20 hours), filtering, and vacuum drying the resultant solid to afford the crystal.

In a preferred embodiment, the weight-to-volume ratio (g/mL) of Compound A to the ester solvent is about 1:(1-10), preferably about 1:5.

In a preferred embodiment, the ester solvent with 2-10 carbon atoms is methyl acetate, ethyl acetate, isopropyl acetate, or ethyl isopropionate.

In a preferred embodiment, the vacuum drying is carried out at about 20-50°C (preferably about 40°C), for about 1-10 hours (preferably 6 hours).

In an embodiment, the present invention provides Crystalline Form III of Compound A monohydrate: 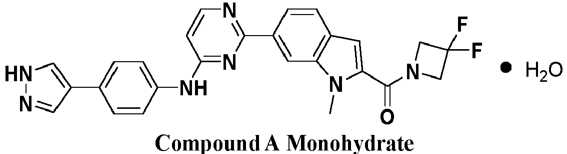 wherein the Crystalline Form III has an XRPD pattern comprising characteristic peaks at diffraction angles (2θ) of about 8.7±0.2°, 12.3±0.2° and 13.8±0.2°.

In a preferred embodiment, the Crystalline Form III has an XRPD pattern comprising characteristic peaks at diffraction angles (2θ) of about 8.7±0.2°, 12.3±0.2°, 13.8±0.2°, 18.5±0.2°, 19.5±0.2°, 21.0±0.2° and 24.5±0.2°.

In a more preferred embodiment, the Crystalline Form III has an XRPD pattern comprising characteristic peaks at diffraction angles (2Θ) of about 8.7±0.2°, 12.3±0.2°, 13.8±0.2°, 16.9±0.2°, 17.6±0.2°, 18.2±0.2°, 18.5±0.2°, 19.5±0.2°, 19.9±0.2°, 21.0±0.2°, 22.5±0.2°, 24.5±0.2° and 24.7±0.2°.

In a more preferred embodiment, the Crystalline Form III has an XRPD pattern comprising peaks at the following diffraction angles (2θ):

| **Peak No.** | **2θ (°) ± 0.2°** | **I%** | **Peak No.** | **2θ (°) ± 0.2°** | **I%** | **Peak No.** | **2θ (°) ± 0.2°** | **I%** |
|---|---|---|---|---|---|---|---|---|
| 1 | 6.1 | 2.8 | 15 | 19.9 | 3.4 | 29 | 28.7 | 0.7 |
| 2 | 8.7 | 8.1 | 16 | 21.0 | 4.3 | 30 | 29.5 | 2.3 |
| 3 | 12.3 | 100 | 17 | 21.5 | 0.8 | 31 | 30.4 | 1.8 |
| 4 | 12.9 | 2 | 18 | 22.2 | 2 | 32 | 30.8 | 1.7 |
| 5 | 13.8 | 8 | 19 | 22.5 | 3.8 | 33 | 31.0 | 1.3 |
| 6 | 15.0 | 1.2 | 20 | 23.3 | 1.4 | 34 | 31.7 | 1.1 |
| 7 | 15.5 | 2.1 | 21 | 23.9 | 1.2 | 35 | 33.6 | 1 |
| 8 | 16.2 | 2 | 22 | 24.5 | 5.2 | 36 | 34.9 | 0.7 |
| 9 | 16.6 | 1.2 | 23 | 24.7 | 4.1 | 37 | 35.8 | 1.2 |
| 10 | 16.9 | 4 | 24 | 25.3 | 2.4 | 38 | 36.4 | 1.3 |
| 11 | 17.6 | 3.3 | 25 | 25.6 | 1.6 | 39 | 37.5 | 2.4 |
| 12 | 18.2 | 3.1 | 26 | 26.3 | 1 | 40 | 38.1 | 0.9 |
| 13 | 18.5 | 4.5 | 27 | 27.4 | 0.9 | 41 | 39.7 | 2.1 |
| 14 | 19.5 | 4.1 | 28 | 28.0 | 0.8 | 42 | 28.7 | 0.7 |

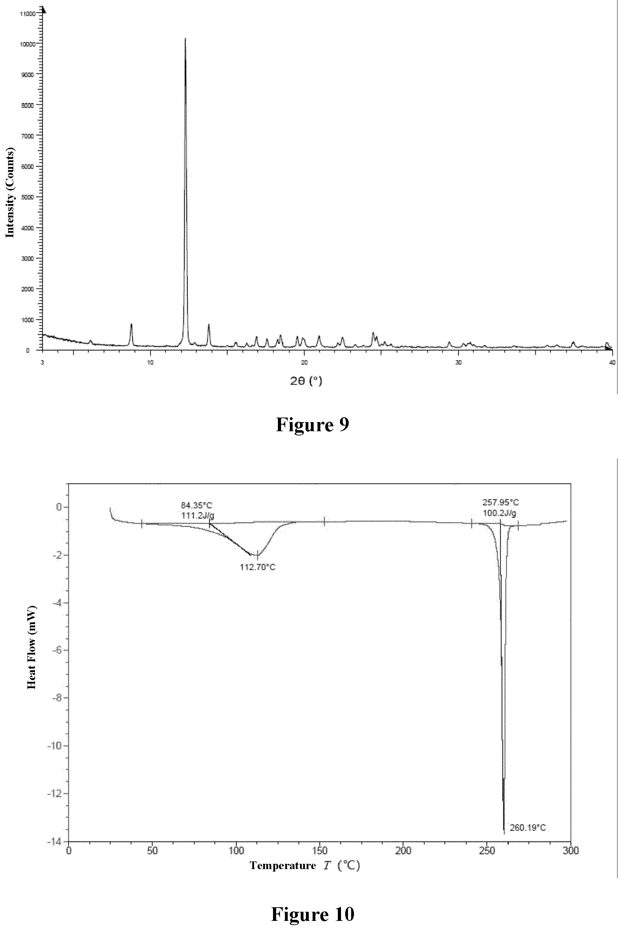

In a more preferred embodiment, the Crystalline Form III has an XRPD pattern comprising peaks at diffraction angles (2Θ) essentially the same as shown in Figure 9. In the most preferred embodiment, the XRPD pattern of the Crystalline Form III is essentially the same as shown in Figure 9.

In a more preferred embodiment, the Crystalline Form III has a DSC graph comprising a broad endothermic peak at about 84-131°C and an endothermic peak at about 260°C.

In a more preferred embodiment, the Crystalline Form III has a DSC graph comprising characteristic peaks essentially the same as shown in Figure 10. In the most preferred embodiment, the DSC graph of the Crystalline Form III is essentially the same as shown in Figure 10.

In a more preferred embodiment, in a thermogravimetric analysis, the Crystalline Form III has a weight loss of about 3.3% when heated to about 150°C.

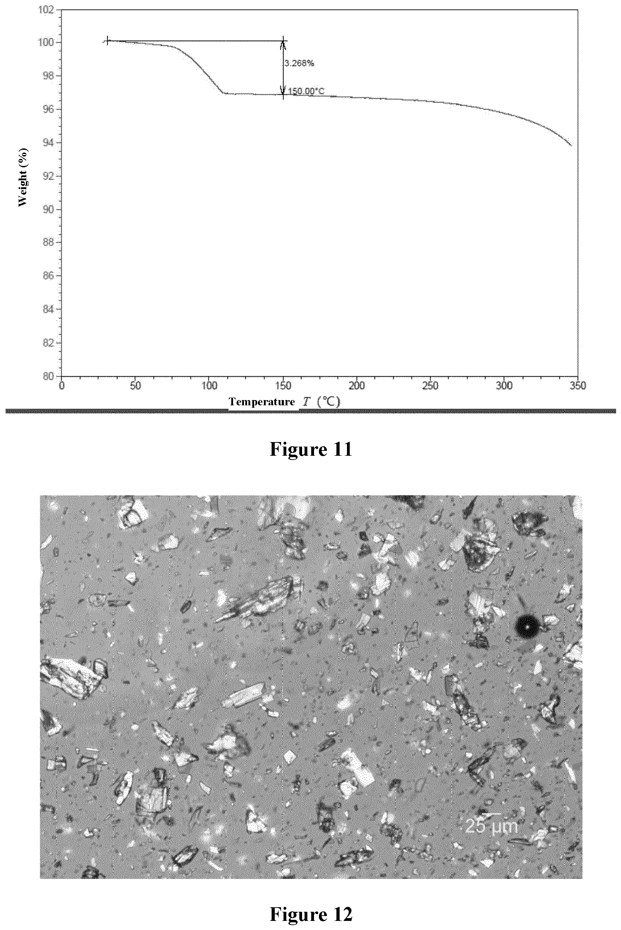

In the most preferred embodiment, the TGA graph of the Crystalline Form III is essentially the same as shown in Figure 11.

In the most preferred embodiment, the scanning electron microscope image of the Crystalline Form III is essentially the same as shown in Figure 12.

In some embodiments, the present invention provides a method for preparing the Crystalline Form III, which comprises stirring Compound A in a mixed solvent comprising an alcohol with 1-10 carbon atoms and water (for example, at about 20-80°C (for example, about 55°C), for about 1-30 hours, preferably about 10-20 hours), filtering, and vacuum drying the resultant solid to afford the crystal.

In a preferred embodiment, the weight-to-volume ratio (g/mL) of Compound A to the mixed solvent is about 1:(1-15), preferably about 1:7.5.

In a preferred embodiment, the alcohol solvent with 1-10 carbon atoms is an alcohol with 1-6 carbon atoms, including but not limited to methanol, ethanol, 1-propanol (n-propanol), 2-propanol (isopropanol), 1-butanol, 2-butanol, and *tert*-butanol.

In a preferred embodiment, the volume ratio of the alcohol with 1-10 carbon atoms to water in the mixed solvent is about (1-5):1, preferably about 2:1.

In a preferred embodiment, the vacuum drying is carried out at about 20-50°C (preferably about 40°C), for about 1-10 hours (preferably 6 hours).

In an embodiment, the present invention provides Crystalline Form IV of Compound A monohydrate: 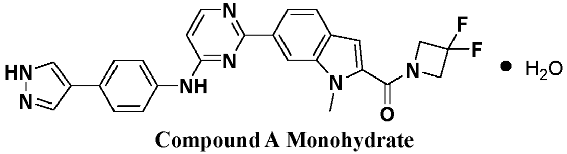 wherein the Crystalline Form IV has an XRPD pattern comprising characteristic peaks at diffraction angles (2Θ) of about 8.9±0.2°, 11.4±0.2° and 17.9±0.2°.

In a preferred embodiment, the Crystalline Form IV has an XRPD pattern comprising characteristic peaks at diffraction angles (2Θ) of about 8.3±0.2°, 8.9±0.2°, 9.2±0.2°, 11.4±0.2°, 15.3±0.2°, 17.9±0.2°, 22.0±0.2° and 26.9±0.2°.

In a more preferred embodiment, the Crystalline Form IV has an XRPD pattern comprising characteristic peaks at diffraction angles (2θ) of about 5.7±0.2°, 8.3±0.2°, 8.9±0.2°, 9.2±0.2°, 10.2±0.2°, 11.4±0.2°, 12.4±0.2°, 15.3±0.2°, 16.4±0.2°, 16.8±0.2°, 17.2±0.2°, 17.4±0.2°, 17.9±0.2°, 18.5±0.2°, 19.6±0.2°, 20.3±0.2°, 20.8±0.2°, 21.3±0.2°, 22.0±0.2°, 22.4±0.2°, 23.0±0.2°, 23.8±0.2°, 24.8±0.2°, 26.1±0.2°, 26.9±0.2°, 27.8±0.2° and 28.7±0.2°.

In a more preferred embodiment, the Crystalline Form IV has an XRPD pattern comprising peaks at the following diffraction angles (2θ):

| **Peak No.** | **2θ (°) ± 0.2°** | **I%** | **Peak No.** | **2θ (°) ± 0.2°** | **I%** | **Peak No.** | **2θ (°) ± 0.2°** | **I%** |
|---|---|---|---|---|---|---|---|---|
| 1 | 5.7 | 18 | 11 | 17.2 | 32.5 | 21 | 23.0 | 21.6 |
| 2 | 8.3 | 39.7 | 12 | 17.4 | 33.4 | 22 | 23.8 | 32.1 |
| 3 | 8.9 | 54.6 | 13 | 17.9 | 66.2 | 23 | 24.8 | 29.4 |
| 4 | 9.2 | 49.9 | 14 | 18.5 | 34 | 24 | 26.1 | 27.6 |
| 5 | 10.2 | 26.2 | 15 | 19.6 | 18.9 | 25 | 26.9 | 36.4 |
| 6 | 11.4 | 100 | 16 | 20.3 | 17.4 | 26 | 27.8 | 18.6 |
| 7 | 12.4 | 25.9 | 17 | 20.8 | 26.5 | 27 | 28.7 | 12.7 |
| 8 | 15.3 | 35.1 | 18 | 21.3 | 20.2 | 28 | 31.8 | 9.2 |
| 9 | 16.4 | 17.3 | 19 | 22.0 | 42.4 | | | |
| 10 | 16.8 | 21.4 | 20 | 22.4 | 24 | | | |

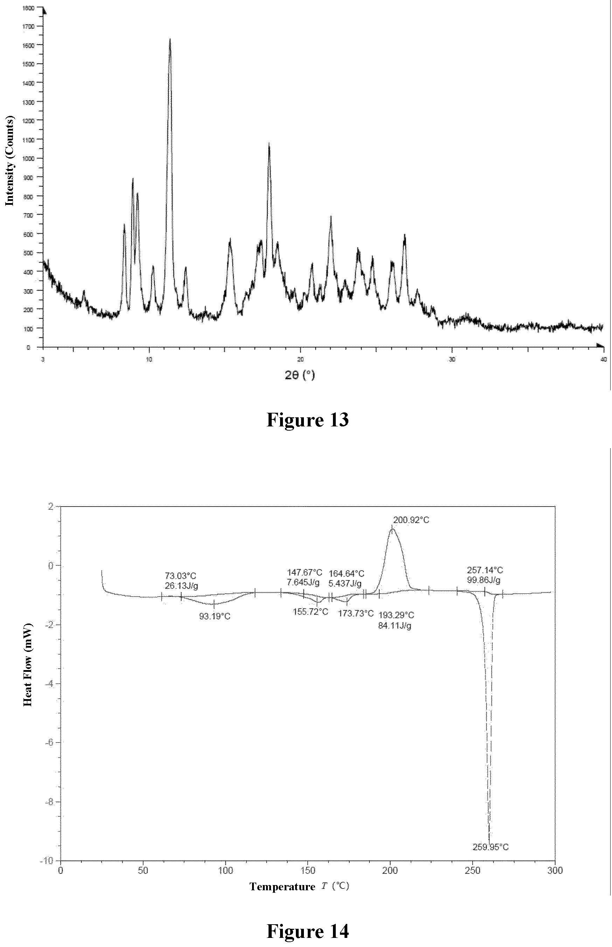

In a more preferred embodiment, the Crystalline Form IV has an XRPD pattern comprising peaks at diffraction angles (2θ) essentially the same as shown in Figure 13. In the most preferred embodiment, the XRPD pattern of the Crystalline Form IV is essentially the same as shown in Figure 13.

In a more preferred embodiment, the Crystalline Form IV has a DSC graph comprising an endothermic peak at about 93°C, an endothermic peak at about 156°C, an endothermic peak at about 174°C, an exothermic peak at about 201°C, and an endothermic peak at about 260°C.

In a more preferred embodiment, the Crystalline Form IV has a DSC graph comprising characteristic peaks essentially the same as shown in Figure 14. In the most preferred embodiment, the DSC graph of the Crystalline Form IV is essentially the same as shown in Figure 14.

In a more preferred embodiment, in a thermogravimetric analysis, the Crystalline Form IV has a weight loss of about 0.8% when heated to about 63°C, and a weight loss of about 3.8% when heated to about63-165°C.

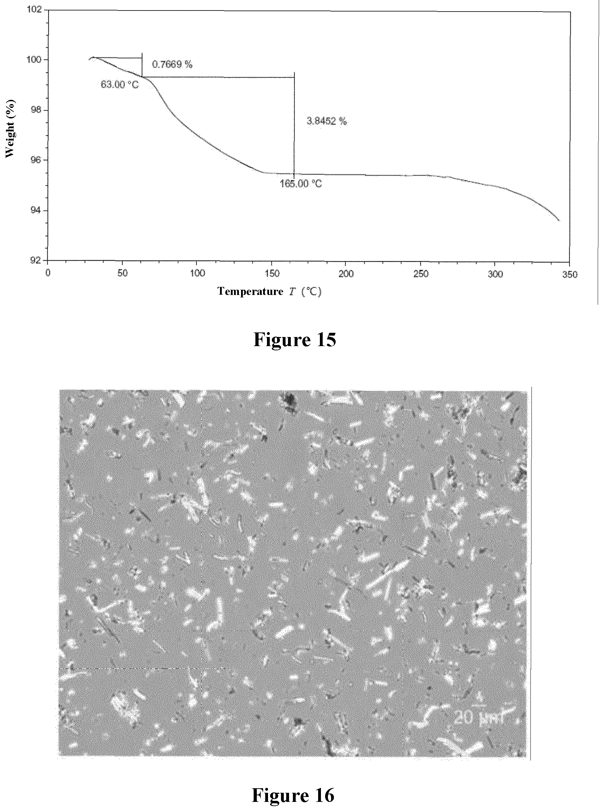

In the most preferred embodiment, the TGA graph of the Crystalline Form IV is essentially the same as shown in Figure 15.

In the most preferred embodiment, the scanning electron microscope image of the Crystalline Form IV is essentially the same as shown in Figure 16.

In some embodiments, the present invention provides a method for preparing the Crystalline Form IV, which comprises stirring Compound A in a mixed solvent comprising a hydrocarbon with 1-10 carbon atoms and an alcohol with 1-10 carbon atoms (for example, at room temperature, for about 1-30 hours, preferably about 10-20 hours), filtering, and vacuum drying the resultant solid to afford the crystal.

In a preferred embodiment, the weight-to-volume ratio (g/mL) of Compound A to the mixed solvent is about 1:(1-10), preferably about 1:5.5.

In a preferred embodiment, the hydrocarbon solvent with 1-10 carbon atoms includes alkanes, halogenated alkanes, alkenes, alkynes, and aromatic hydrocarbons, specifically including but not limited to dichloromethane, trichloromethane (chloroform), n-hexane, n-heptane, and toluene.

In a preferred embodiment, the alcohol solvent with 1-10 carbon atoms is an alcohol with 1-6 carbon atoms, which includes but is not limited to methanol, ethanol, 1-propanol (n-propanol), 2-propanol (isopropanol), 1-butanol, 2-butanol, and *tert*-butanol.

In a preferred embodiment, the volume ratio of the hydrocarbon with 1-10 carbon atoms to the alcohol with 1-10 carbon atoms in the mixed solvent is about (1-15): 1, preferably about 10:1.

In a preferred embodiment, the vacuum drying is carried out at about 20-50°C (preferably about 40°C), for about 1-10 hours (preferably 6 hours).

In an embodiment, the present invention provides Crystalline Form V of Compound A monohydrate: 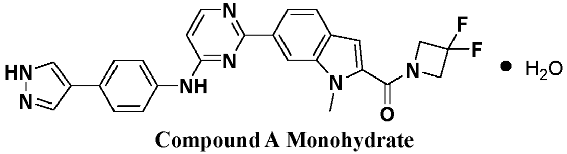 wherein the Crystalline Form V has an XRPD pattern comprising characteristic peaks at diffraction angles (2θ) of about 8.7±0.2°, 12.2±0.2° and 24.5±0.2°.

In a preferred embodiment, the Crystalline Form V has an XRPD pattern comprising characteristic peaks at diffraction angles (2θ) of about 8.7±0.2°, 12.2±0.2°, 13.7±0.2°, 16.9±0.2°, 20.0±0.2°, 20.9±0.2° and 24.5±0.2°.

In a more preferred embodiment, the Crystalline Form V has an XRPD pattern comprising characteristic peaks at diffraction angles (2θ) of about 8.7±0.2°, 12.2±0.2°, 12.8±0.2°, 13.7±0.2°, 15.5±0.2°, 16.3±0.2°, 16.9±0.2°, 17.5±0.2°, 20.0±0.2°, 20.9±0.2°, 22.2±0.2°, 22.5±0.2°, 24.5±0.2°, 25.2±0.2°, 29.4±0.2° and 30.3±0.2°.

In a more preferred embodiment, the Crystalline Form V has an XRPD pattern comprising peaks at the following diffraction angles (2θ):

| **Peak No.** | **2θ (°) ± 0.2°** | **I%** | **Peak No.** | **2θ (°) ± 0.2°** | **I%** | **Peak No.** | **2θ (°) ± 0.2°** | **I%** |
|---|---|---|---|---|---|---|---|---|
| 1 | 6.0 | 1.1 | 12 | 17.5 | 15.5 | 23 | 25.6 | 2.6 |
| 2 | 6.9 | 3.3 | 13 | 18.3 | 9.3 | 24 | 26.4 | 2.6 |
| 3 | 8.7 | 100 | 14 | 19.5 | 8.4 | 25 | 29.4 | 11.9 |
| 4 | 11.1 | 1 | 15 | 20.0 | 29.9 | 26 | 30.3 | 13.4 |
| 5 | 12.2 | 66.3 | 16 | 20.9 | 43.2 | 27 | 32.8 | 1.1 |
| 6 | 12.8 | 19.7 | 17 | 22.2 | 13.4 | 28 | 33.8 | 1.1 |
| 7 | 13.7 | 21.1 | 18 | 22.5 | 12.9 | 29 | 35.8 | 2.9 |
| 8 | 15.0 | 5.5 | 19 | 23.3 | 3 | 30 | 38.1 | 1.1 |
| 9 | 15.5 | 14.2 | 20 | 23.9 | 4.8 | 31 | 39.7 | 2 |
| 10 | 16.3 | 17.4 | 21 | 24.5 | 59.3 | 32 | 41.4 | 0.6 |
| 11 | 16.9 | 49 | 22 | 25.2 | 16.7 | 33 | 46.1 | 0.9 |

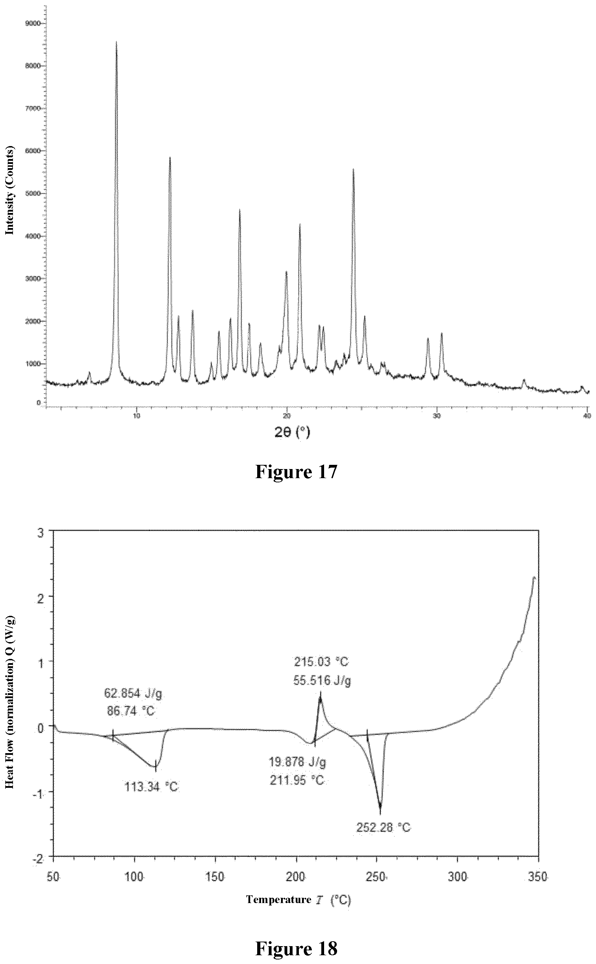

In a more preferred embodiment, the Crystalline Form V has an XRPD pattern comprising peaks at diffraction angles (2Θ) essentially the same as shown in Figure 17. In the most preferred embodiment, the XRPD pattern of the Crystalline Form V is essentially the same as shown in Figure 17.

In a more preferred embodiment, the Crystalline Form V has a DSC graph comprising an endothermic peak at about 113°C, an exothermic peak at about 215°C, and an endothermic peak at about 252°C.

In a more preferred embodiment, the Crystalline Form V has a DSC graph comprising characteristic peaks essentially the same as shown in Figure 18. In the most preferred embodiment, the DSC graph of the Crystalline Form V is essentially the same as shown in Figure 18.

In a more preferred embodiment, in a thermogravimetric analysis, the Crystalline Form V has a weight loss of about 3.5% when heated to about 130°C.

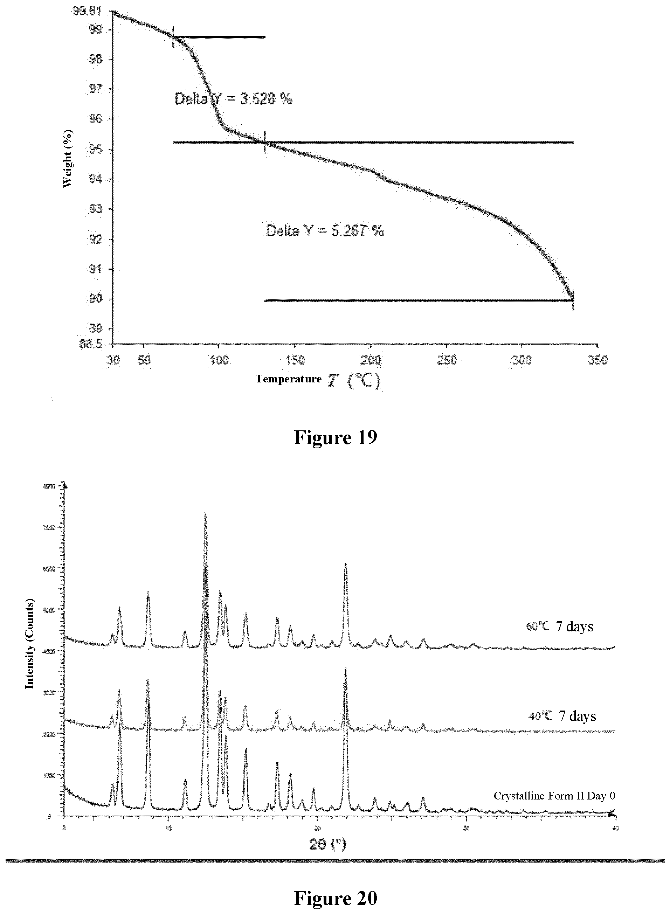

In the most preferred embodiment, the TGA graph of the Crystalline Form V is essentially the same as shown in Figure 19.

In some embodiments, the present invention provides a method for preparing the Crystalline Form V, which comprises adding an acid addition salt of Compound A to a mixed solvent comprising an alcohol with 1-10 carbon atoms and water, then adding a base, and stirring the mixture (for example, at room temperature, for about 1-15 hours, preferably about 1-10 hours), filtering, and vacuum drying the resultant solid to afford the crystal.

In a preferred embodiment, the weight-to-volume ratio (g/mL) of the acid addition salt of Compound A (preferably a hydrochloride salt of Compound A) to the mixed solvent is about 1:(1-10), preferably about 1:5.5.

In a preferred embodiment, the molar ratio of the acid addition salt of Compound A to the base is about 1:(1-5), preferably about 1:(1-2).

In a preferred embodiment, the base is an organic base, such as triethylamine.

In a preferred embodiment, the alcohol solvent with 1-10 carbon atoms is an alcohol with 1-6 carbon atoms, which include but are not limited to methanol, ethanol, 1-propanol (n-propanol), 2-propanol (isopropanol), 1-butanol, 2-butanol, and *tert*-butanol.

In a preferred embodiment, the volume ratio of the alcohol with 1-10 carbon atoms to water in the mixed solvent is about (1-15):1, preferably about 10:1.

In a preferred embodiment, the vacuum drying is carried out at about 20-50°C (preferably about 40°C), for about 1-10 hours (preferably 4 hours).

### Pharmaceutical composition, therapeutic method and use thereof

In some embodiments, the present invention provides a pharmaceutical composition comprising any one or more of Crystalline Forms I, II, III, IV and V of the present invention and one or more pharmaceutically acceptable carriers.

In some embodiments, the present invention provides use of any one or more of Crystalline Forms I, II, III, IV and V of the present invention in the manufacture of a medicament as a Rho-associated protein kinase (ROCK) inhibitor, preferably a selective ROCK2 inhibitor.

In some embodiments, the present invention provides any one or more of Crystalline Forms I, II, III, IV and V of the present invention for use as a Rho-associated protein kinase (ROCK) inhibitor, preferably a selective ROCK2 inhibitor.

In some embodiments, the present invention provides a method for the prophylaxis or treatment of a disease mediated by the Rho-associated protein kinase (ROCK), comprising administering to a subject in need thereof, preferably a mammal, a prophylactically or therapeutically effective amount of any one or more of Crystalline Forms I, II, III, IV and V of the present invention.

In some embodiments, the disease mediated by the Rho-associated protein kinase (ROCK) includes an autoimmune disorder (comprising rheumatoid arthritis, systemic lupus erythematosus (SLE; lupus), psoriasis, Crohn's disease, atopic dermatitis, eczema, or graft-versus-host disease (GVHD)); a cardiovascular disorder (comprising hypertension, atherosclerosis, restenosis, cardiac hypertrophy, cerebral ischemia, cerebral vasospasm, or erectile dysfunction); inflammation (comprising asthma, cardiovascular inflammation, ulcerative colitis, or renal inflammation); a central nervous system disorder (comprising neuronal degeneration or spinal cord injury; and the central nervous system disorder is preferably Huntington's disease, Parkinson's disease, Alzheimer's disease, Amyotrophic lateral sclerosis (ALS), or multiple sclerosis); an arterial thrombotic disorder (comprising platelet aggregation, or leukocyte aggregation); a fibrotic disorder (comprising liver fibrosis, lung fibrosis, or kidney fibrosis); a neoplastic disease (comprising a lymphoma, carcinoma (e.g., squamous cell cancer, small-cell lung cancer, pituitary cancer, esophageal cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, bladder cancer, liver cancer, breast cancer, colon cancer, colorectal cancer, endometrial or uterine carcinoma, salivary gland carcinoma, kidney cancer, prostate cancer, vulval cancer, thyroid cancer, brain cancer, endometrial cancer, testis cancer, cholangiocarcinoma, gallbladder carcinoma, gastric cancer, melanoma, or head and neck cancer), leukemia, astrocytoma, soft tissue sarcoma, sarcoma, or blastoma); a metabolic syndrome; insulin resistance; hyperinsulinemia; type 2 diabetes; glucose intolerance; osteoporosis; an ocular disorder (comprising ocular hypertension, age related macular degeneration (AMD), choroidal neovascularization (CNV), diabetic macular edema (DME), iris neovascularization, uveitis, glaucoma (comprising primary open-angle glaucoma, acute angle-closure glaucoma, pigmentary glaucoma, congenital glaucoma, normal tension glaucoma, secondary glaucoma or neo vascular glaucoma), or retinitis of prematurity (ROP)).

In some embodiments, the disease mediated by the Rho-associated protein kinase (ROCK) includes lupus nephritis, atherosclerosis, rheumatoid arthritis (RA), hemangioma, angiofibroma, lung fibrosis, psoriasis, corneal graft rejection, insulin-dependent diabetes mellitus, multiple sclerosis, myasthenia gravis, Chron's disease, autoimmune nephritis, primary biliary cirrhosis, acute pancreatitis, allograph rejection, allergic inflammation, contact dermatitis, delayed hypersensitivity, inflammatory bowel disease, septic shock, osteoporosis, osteoarthritis, neuronal inflammation, Osier-Weber syndrome, restenosis, fungal infection, parasitic infection, and viral infection.

As used herein, the term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g., Remington's Pharmaceutical Sciences (1990).

The composition of the present invention can act systemically and/or topically. To this end, it can be administered through a suitable route, such as through injection, intravenous, intraarterial, subcutaneous, intraperitoneal, intramuscular, or transdermal administration, or administered via oral, buccal, nasal, transmucosal, topical, as an ophthalmic formulation, or via inhalation.

For these routes of administration, the composition of the present invention can be administered in a suitable dosage form.

The dosage form may be solid, semi-solid, liquid, or gas formulations, specifically including, but not limited to, tablets, capsules, powders, granules, lozenges, hard candies, powders, sprays, creams, salves, suppositories, gels, pastes, lotions, ointments, aqueous suspensions, injectable solutions, suspensions, elixirs, and syrups.

The pharmaceutical composition of the present invention may be manufactured by any process well known in the art, e.g., by means of mixing, dissolving, granulating, dragee-making, levigating, emulsifying, lyophilizing processes, or the like.

As used herein, the term "therapeutically effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, and 25 mg, *etc.*

Unless otherwise indicated, the term "treating" or "treatment", as used herein, means reversing, alleviating, inhibiting the progress of, or preventing a disorder, condition, or disease to which such term applies, or one or more symptoms of such disorder, condition, or disease.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

### Examples

The present invention is explained in more detail below with reference to the examples, which are only used to illustrate the technical solutions of the present invention, and are not intended to limit the scope thereof. Those skilled in the art may make some non-essential improvements and adjustments, which still fall within the scope of the present invention.

Unless otherwise specified, the starting materials and reagents employed in the following Examples are all commercially available products or can be prepared through known methods.

The detection instruments and conditions used in the following examples are as follows:
(1) X-ray powder diffraction (XRPD)
   Instrument Model: Bruker D8 advance, equipped with a LynxEye detector
   Test conditions: the anode target material was copper, the light pipe was set to (40KV 40mA), the 2Θ scan angle for the sample was from 3° to 40°, and scan step was 0.02°.
(2) differential scanning calorimetry analysis (DSC)
   Instrument Model: TA Discovery DSC 250 (TA Instruments, US)
   Test conditions: the heating rate was 10°C/min, and dry nitrogen was used as the purge gas.
(3) thermogravimetric analysis (TGA)
   Instrument Model: Discovery TGA 55 (TA Instruments, US)
   Test conditions: automatic weighing in the heating furnace, the heating rate was 10°C/min, and dry nitrogen was used as the purge gas.
(4) polarizing microscope analysis (PLM)
   Instrument Model: Polarizing Microscope ECLIPSE LV100POL (Nikon, JPN)

### Example 1: Preparation of (6-(4-((4-(1H-pyrazol-4-yl)phenyl)amino)pyrimidin-2-yl)-1-methyl-1H-indol-2-yl)(3,3-difluoroazetidi n-1-yl)methanone (Compound A) (according to PCT/CN2018/093713, which is incorporated herein in its entirety by reference)

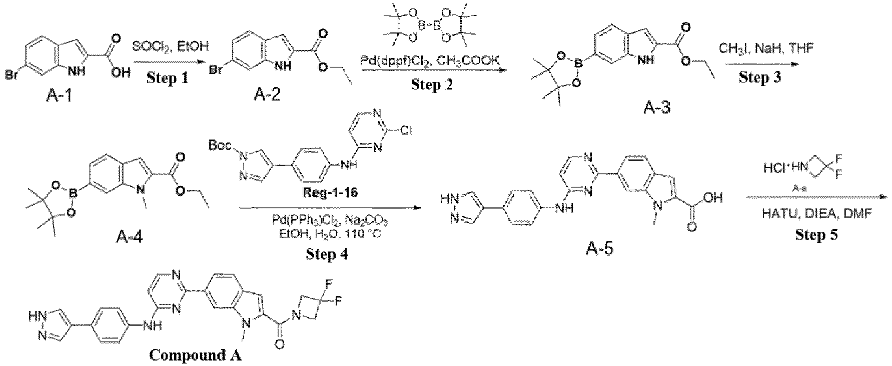

### Step 1:

Compound **A-1** (20 g, 83.31 mmol) and ethanol (200 mL) were added to a 500 mL flask, thionyl chloride (19.82 g, 166.63 mmol) was added, and then the reaction was performed at 60°C for 3 hours. Thin layer chromatography (petroleum ether / ethyl acetate =10:1) assay indicated the reaction was complete. The reaction solution was concentrated to afford a crude product, which was dissolved in dichloromethane (500 mL), and the resulting solution was washed twice with a saturated aqueous solution of sodium bicarbonate (150 mL each). The organic phase was washed with saturated brine, then dried over anhydrous sodium sulfate, filtered, and concentrated to afford compound **A-2** (21 g, brown solid, yield: 94.01%). MS *m*/*z* (ESI): 266.1; 268.1 [M-H].

### Step 2:

Compound **A-2** (21 g, 78.33 mmol) and bis(pinacolato)diboron (26.85 g, 105.74 mmol) were dissolved in 1,4-dioxane (200 mL), potassium acetate (23.06 g, 234.98 mmol) and Pd(dppf)Cl₂ (3.24 g, 3.91 mmol) were added, purge with argon was performed for 3 times, and the reaction was placed in an oil bath at 80 °C overnight. LC-MS indicated the reaction was complete. The reaction solution was cooled to room temperature, concentrated under reduced pressure, and the residue was purified by column chromatography (petroleum ether / ethyl acetate = 100:1 to 5:1) to afford compound A-3 (17.5 g, white solid, yield: 70.89%).

¹H NMR (400 MHz, CDCl₃) δ 8.92 (s, 1H), 7.92 (s, 1H), 7.69 (d, *J* = 8.1 Hz, 1H), 7.57 (d, *J* = 8.1 Hz, 1H), 7.22 - 7.18 (m, 1H), 4.42 (q, *J* = 7.1 Hz, 2H), 1.42 (t, *J* = 7.1 Hz, 3H), 1.37 (s, 12H). MS *m*/*z* (ESI): 316.2 [M+H].

### Step 3:

Compound **A-3** (10.0 g, 31.8 mmol) was dissolved in tetrahydrofuran (250 mL), sodium hydride (1.91 g, 47.8 mmol) was added under ice bath cooling, and then the reaction was performed for 30 minutes. Iodomethane (13.5 g, 95.4 mmol) was slowly added to the reaction solution, and the reaction was performed at room temperature overnight. Thin layer chromatography (petroleum ether / ethyl acetate = 5:1) indicated the reaction was complete. The reaction solution was quenched with water (100 mL), and extracted with ethyl acetate (150 mL x 2). The combined organic phases were washed sequentially with a saturated aqueous solution of ammonium chloride (200 mL x 2) and saturated brine (300 mL x 2), dried over anhydrous sodium sulfate, filtered, and concentrated. The crude product was separated and purified by column chromatography (petroleum ether / ethyl acetate = 15:1) to afford compound A-4 (6.5 g, yellow solid, yield: 62.5%).

¹H NMR (400 MHz, CDCl₃) δ 7.91 (s, 1H), 7.68 - 7.65 (m, 1H), 7.57 (d, *J* = 8.0 Hz, 1H), 7.27 (s, 1H), 4.12 (s, 3H), 3.91 (s, 3H), 1.38 (s, 12H).

### Step 4:

Compound **Reg-1-16** (1.00 g, 2.70 mmol) and **A-4** (1.33 g, 4.04 mmol) were dissolved in a mixed solution of ethanol/water (8:1) (120 mL), sodium carbonate (572 mg, 5.40 mmol) and Pd(PPh₃)Cl₂ (189 mg, 0.27 mmol) were added, purge with argon was performed for 3 times, and the reaction was placed in an oil bath at 110 °C overnight. LC-MS indicated the reaction was complete. The reaction solution was cooled to room temperature, and concentrated under reduced pressure. The residue was diluted with water (30 mL), and the pH was adjusted to 1 with 6N HCl. A large amount of solid precipitated, and was filtered. The solide was wahsed with methanol to afford compound A-5 (900 mg, yellow solid, crude product).

¹H NMR (400 MHz, DMSO-*d₆*) *δ* 11.54 (s, 1H), 8.77 (s, 1H), 8.38 (d, *J* = 7.2 Hz, 1H), 8.14 (s, 2H), 8.03 (d, *J* = 8.8 Hz, 1H), 7.92 (d, *J* = 8.4 Hz, 1H), 7.80 - 7.69 (m, 4H), 7.33 (s, 1H), 7.07 (d, *J* = 8.0 Hz, 1H), 4.16 (s, 3H).

### Step 5:

Compound **A-5** (300 mg, 0.73 mmol) was dissolved in N,N-dimethylformamide (6 mL), HATU (335 mg, 0.88 mmol) and DIEA (377 mg, 2.92 mmol) were added, and the reaction was performed at room temperature for 30 min. Then, compound **A-a** (114 mg, 0.88 mmol) was added, and the reaction was continued at room temperature for 2 hours. LC-MS indicated the reaction was complete. The reaction solution was concentrated under reduced pressure, and the crude product was purified by high performance liquid chromatography to afford Compound **A** (185 mg, yellow solid, yield: 52.1%).

¹H NMR (400 MHz, DMSO-*d₆*) *δ* 10.63 (s, 1H), 8.52 (s, 1H), 8.41 (d, *J* = 6.4 Hz, 1H), 8.09 (s, 2H), 8.06 (dd, *J* = 8.4, 1.2 Hz, 1H), 7.83 (d, *J* = 8.4 Hz, 1H), 7.77 (d, *J* = 8.0 Hz, 2H), 7.72 (d, *J* = 8.4 Hz, 2H), 7.13 (s, 1H), 6.86 (d, *J* = 6.4 Hz, 1H), 4.91 (s, 2H), 4.57 (s, 2H), 4.05 (s, 3H). MS *m*/*z* (ESI): 486.2 [M+H].

### Example 2: Preparation of Crystalline Form I of Compound A Monohydrate

Compound A (2.0 g) was added to a solution of dichloromethane (5 mL), DMF (1 mL), and water (1 mL) and stirred at room temperature for 18h. After filtration, the solid was collected, the filter cake was rinsed with water (2 mL), and then vacuum dried at 40°C for 6h to obtain the crystal (1.0 g, yield 48.2%). The XRPD pattern was determined by X-ray powder diffraction and is shown in Figure 1; the DSC graph is shown in Figure 2 and the TGA graph in Figure 3 as analyzed by DSC and TGA; the sample was observed under a scanning electron microscope and the crystal morphology is shown in Figure 4.

### Example 3: Preparation of Crystalline Form II of Compound A Anhydrous

Compound A (2.0 g) was added to ethyl acetate (10 mL) and stirred at 55°C for 18h. After filtration, the solid was collected and vacuum dried at 40°C for 6h to obtain the crystal (1.4 g, yield 70.0%). The XRPD pattern was determined by X-ray powder diffraction and is shown in Figure 5; the DSC graph is shown in Figure 6 and the TGA graph in Figure 7 as analyzed by DSC and TGA; the sample was observed under a scanning electron microscope and the crystal morphology is shown in Figure 8.

### Example 4: Preparation of Crystalline Form III of Compound A Monohydrate

Compound A (2.0 g) was added to a mixture of methanol (10 mL) and water (5 mL), and stirred at 55°C for 18h. After filtration, the solid was collected and vacuum dried at 40°C for 6h to obtain the crystal (1.2 g, yield 57.8%). The XRPD pattern was determined by X-ray powder diffraction and is shown in Figure 9; the DSC graph is shown in Figure 10 and the TGA graph in Figure 11 as analyzed by DSC and TGA; the sample was observed under a scanning electron microscope and the crystal morphology is shown in Figure 12.

### Example 5: Preparation of Crystalline Form IV of Compound A Monohydrate

Compound A (2.0 g) was added to a mixture of dichloromethane (10 mL) and methanol (1 mL), stirred at room temperature for 18h. After filtration, the solid was collected and vacuum dried at 40°C for 6h to obtain the crystal (1.3 g, yield 62.7%). The XRPD pattern was determined by X-ray powder diffraction and is shown in Figure 13; the DSC graph is shown in Figure 14 and the TGA graph in Figure 15 as analyzed by DSC and TGA; the sample was observed under a scanning electron microscope and the crystal morphology is shown in Figure 16.

### Example 6: Preparation of Crystalline Form V of Compound A Monohydrate

Compound A (3.7 g) was added to a flask and acetone (250 mL) was added. The reaction solution was heated to 50°C and stirred for 10 min until the solid was completely dissolved. Water (26.7 mL) was added and stirred until the solution was clear. Concentrated hydrochloric acid (0.641 mL) was then added, causing a large amount of solid to precipitate. The mixture was maintained at 50°C and stirred for 18h, then filtered to collect the solid, which was vacuum dried at 50°C for 8h to obtain the hydrochloride salt of compound A (2.7 g, yield 67.9%).

The hydrochloride salt of compound A (2.0 g) was added to anhydrous ethanol (10 mL) and water (1 mL), and triethylamine (0.4 g) was added. The mixture was stirred at room temperature for 5h, then filtered to collect the solid, which was vacuum dried at 40°C for 4h to obtain the crystal (1.4 g, yield 72.6%). The XRPD pattern was determined by X-ray powder diffraction and is shown in Figure 17; the DSC graph is shown in Figure 18 and the TGA graph in Figure 19 as analyzed by DSC and TGA.

### Experimental Examples

### Experimental Example 1: Solid Stability Test

Two samples of Crystalline Form II of Compound A anhydrate were placed in environments of 40°C/75% RH and 60°C/90% RH for 7 days. XRPD analysis showed no change in the crystalline form of the samples, indicating excellent stability. The XRPD pattern comparison is shown in Figure 20.

### Experimental Example 2: Solid Heat Stability Test

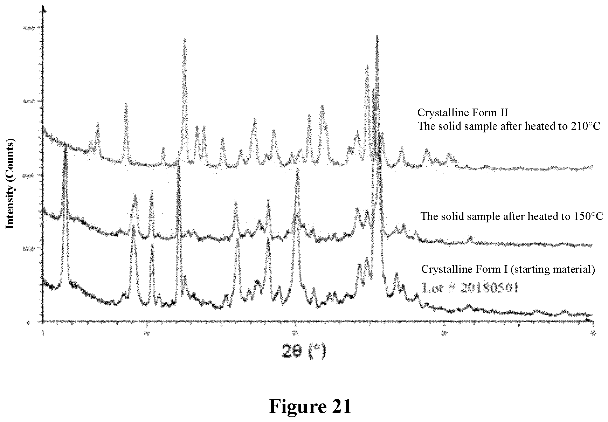

In a differential scanning calorimeter, samples of Crystalline Form I of Compound A monohydrate were heated at a rate of 10°C/min to 150°C and 210°C, respectively, and then analyzed by X-ray powder diffraction. The XRPD patterns of the starting and heat-treated samples are shown in Figure 21. After heated to 210°C, the crystalline form of the sample was converted to crystalline form II of Compound A anhydrous, whereas the sample remained unchanged after heated to 150°C.

### Experimental Example 3: Solid Hygroscopicity Test

100 mg of Crystalline Form II of Compound A anhydrate and 100 mg of Crystalline Form I of Compound A monohydrate were precisely weighed. The two samples were placed at 25°C±1°C and 80%±2% relative humidity for 24 hours. The weight of the samples was then measured and the percentage weight increase was calculated.

According to the calculation, the weight increase percentage for Crystalline Form II of Compound A anhydrate was found to be 0.69%, and for Crystalline Form I of Compound A monohydrate it was 3.62%. The results indicate that Crystalline Form II of Compound A anhydrate has a lower hygroscopicity compared to Crystalline Form I of Compound A monohydrate.

Various modifications to the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. Crystalline Form I of Compound A monohydrate: 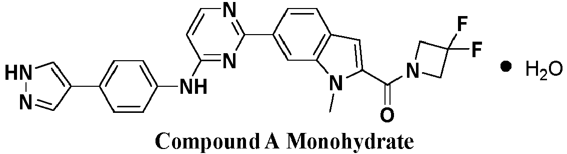 wherein the Crystalline Form I has an XRPD pattern comprising characteristic peaks at diffraction angles (2Θ) of about 4.5±0.2°, 12.2±0.2°, 20.1±0.2°, 25.3±0.2° and 25.5±0.2°; preferably comprising characteristic peaks at diffraction angles (2Θ) of about 4.5±0.2°, 9.1±0.2°, 10.3±0.2°, 12.2±0.2°, 16.1±0.2°, 18.2±0.2°, 20.1±0.2°, 25.3±0.2° and 25.5±0.2°; and most preferably comprising characteristic peaks at diffraction angles (2Θ) of about 4.5±0.2°, 9.1±0.2°, 10.3±0.2°, 12.2±0.2°, 12.5±0.2°, 16.1±0.2°, 16.9±0.2°, 17.4±0.2°, 18.2±0.2°, 18.9±0.2°, 20.1±0.2°, 20.5±0.2°, 21.2±0.2°, 22.6±0.2°, 24.3±0.2°, 24.8±0.2°, 25.3±0.2°, 25.5±0.2°, 26.8±0.2° and 27.2±0.2°.

2. Crystalline Form II of Compound A anhydrate: 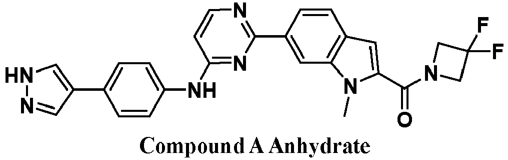 wherein the Crystalline Form II has an XRPD pattern comprising characteristic peaks at diffraction angles (2θ) of about 8.6±0.2°, 12.5±0.2° and 21.9±0.2°; preferably comprising characteristic peaks at diffraction angles (2θ) of about 6.7±0.2°, 8.6±0.2°, 12.5±0.2°, 13.5±0.2°, 13.8±0.2°, 21.9±0.2° and 24.9±0.2°; and most preferably comprising characteristic peaks at diffraction angles (2θ) of about 6.2±0.2°, 6.7±0.2°, 8.6±0.2°, 11.1±0.2°, 12.5±0.2°, 13.5±0.2°, 13.8±0.2°, 15.2±0.2°, 17.3±0.2°, 18.2±0.2°, 18.6±0.2°, 21.0±0.2°, 21.9±0.2°, 24.3±0.2°, 24.9±0.2° and 25.9±0.2°.

3. Crystalline Form III of Compound A monohydrate: 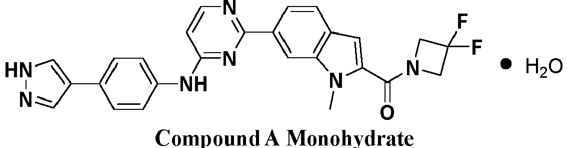 wherein the Crystalline Form III has an XRPD pattern comprising characteristic peaks at diffraction angles (2θ) of about 8.7±0.2°, 12.3±0.2° and 13.8±0.2°; preferably comprising characteristic peaks at diffraction angles (2θ) of about 8.7±0.2°, 12.3±0.2°, 13.8±0.2°, 18.5±0.2°, 19.5±0.2°, 21.0±0.2° and 24.5±0.2°; and most preferably comprising characteristic peaks at diffraction angles (2θ) of about 8.7±0.2°, 12.3±0.2°, 13.8±0.2°, 16.9±0.2°, 17.6±0.2°, 18.2±0.2°, 18.5±0.2°, 19.5±0.2°, 19.9±0.2°, 21.0±0.2°, 22.5±0.2°, 24.5±0.2° and 24.7±0.2°.

4. Crystalline Form IV of Compound A monohydrate: 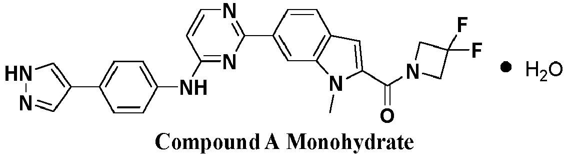 wherein the Crystalline Form IV has an XRPD pattern comprising characteristic peaks at diffraction angles (2θ) of about 8.9±0.2°, 11.4±0.2° and 17.9±0.2°; preferably, the Crystalline Form IV has an XRPD pattern comprising characteristic peaks at diffraction angles (2θ) of about 8.3±0.2°, 8.9±0.2°, 9.2±0.2°, 11.4±0.2°, 15.3±0.2°, 17.9±0.2°, 22.0±0.2° and 26.9±0.2°; and most preferably, the Crystalline Form IV has an XRPD pattern comprising characteristic peaks at diffraction angles (2θ) of about 5.7±0.2°, 8.3±0.2°, 8.9±0.2°, 9.2±0.2°, 10.2±0.2°, 11.4±0.2°, 12.4±0.2°, 15.3±0.2°, 16.4±0.2°, 16.8±0.2°, 17.2±0.2°, 17.4±0.2°, 17.9±0.2°, 18.5±0.2°, 19.6±0.2°, 20.3±0.2°, 20.8±0.2°, 21.3±0.2°, 22.0±0.2°, 22.4±0.2°, 23.0±0.2°, 23.8±0.2°, 24.8±0.2°, 26.1±0.2°, 26.9±0.2°, 27.8±0.2° and 28.7±0.2°.

5. Crystalline Form V of Compound A monohydrate: 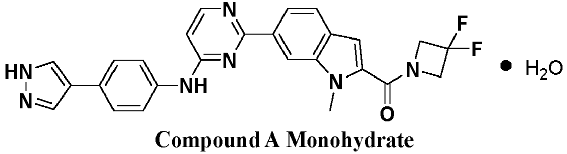 wherein the Crystalline Form V has an XRPD pattern comprising characteristic peaks at diffraction angles (2Θ) of about 8.7±0.2°, 12.2±0.2° and 24.5±0.2°; preferably comprising characteristic peaks at diffraction angles (2θ) of about 8.7±0.2°, 12.2±0.2°, 13.7±0.2°, 16.9±0.2°, 20.0±0.2°, 20.9±0.2° and 24.5±0.2°; and most preferably comprising characteristic peaks at diffraction angles (2θ) of about 8.7±0.2°, 12.2±0.2°, 12.8±0.2°, 13.7±0.2°, 15.5±0.2°, 16.3±0.2°, 16.9±0.2°, 17.5±0.2°, 20.0±0.2°, 20.9±0.2°, 22.2±0.2°, 22.5±0.2°, 24.5±0.2°, 25.2±0.2°, 29.4±0.2° and 30.3±0.2°.

6. A pharmaceutical composition comprising any one or more of the Crystalline Form I according to claim 1, the Crystalline Form II according to claim 2, the Crystalline Form III according to claim 3, the Crystalline Form IV according to claim 4, and the Crystalline Form V according to claim 5, and one or more pharmaceutically acceptable carriers.

7. Use of any one or more of the Crystalline Form I according to claim 1, the Crystalline Form II according to claim 2, the Crystalline Form III according to claim 3, the Crystalline Form IV according to claim 4, and the Crystalline Form V according to claim 5 in the manufacture of a medicament as a Rho-associated protein kinase (ROCK) inhibitor, preferably a selective ROCK2 inhibitor.

8. The use according to claim 7, wherein the medicament is for the prophylaxis or treatment of a disease mediated by the Rho-associated protein kinase (ROCK), including an autoimmune disorder (comprising rheumatoid arthritis, systemic lupus erythematosus (SLE; lupus), psoriasis, Crohn's disease, atopic dermatitis, eczema, or graft-versus-host disease (GVHD)); a cardiovascular disorder (comprising hypertension, atherosclerosis, restenosis, cardiac hypertrophy, cerebral ischemia, cerebral vasospasm, or erectile dysfunction); inflammation (comprising asthma, cardiovascular inflammation, ulcerative colitis, or renal inflammation); a central nervous system disorder (comprising neuronal degeneration or spinal cord injury; and the central nervous system disorder is preferably Huntington's disease, Parkinson's disease, Alzheimer's disease, Amyotrophic lateral sclerosis (ALS), or multiple sclerosis); an arterial thrombotic disorder (comprising platelet aggregation, or leukocyte aggregation); a fibrotic disorder (comprising liver fibrosis, lung fibrosis, or kidney fibrosis); a neoplastic disease (comprising a lymphoma, carcinoma (e.g., squamous cell cancer, small-cell lung cancer, pituitary cancer, esophageal cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, bladder cancer, liver cancer, breast cancer, colon cancer, colorectal cancer, endometrial or uterine carcinoma, salivary gland carcinoma, kidney cancer, prostate cancer, vulval cancer, thyroid cancer, brain cancer, endometrial cancer, testis cancer, cholangiocarcinoma, gallbladder carcinoma, gastric cancer, melanoma, or head and neck cancer), leukemia, astrocytoma, soft tissue sarcoma, sarcoma, or blastoma); a metabolic syndrome; insulin resistance; hyperinsulinemia; type 2 diabetes; glucose intolerance; osteoporosis; an ocular disorder (comprising ocular hypertension, age related macular degeneration (AMD), choroidal neovascularization (CNV), diabetic macular edema (DME), iris neovascularization, uveitis, glaucoma (comprising primary open-angle glaucoma, acute angle-closure glaucoma, pigmentary glaucoma, congenital glaucoma, normal tension glaucoma, secondary glaucoma or neo vascular glaucoma), or retinitis of prematurity (ROP)).

9. The use according to claim 7, wherein the medicament is for the prophylaxis or treatment of a disease mediated by the Rho-associated protein kinase (ROCK), including lupus nephritis, atherosclerosis, rheumatoid arthritis (RA), hemangioma, angiofibroma, lung fibrosis, psoriasis, corneal graft rejection, insulin-dependent diabetes mellitus, multiple sclerosis, myasthenia gravis, Chron's disease, autoimmune nephritis, primary biliary cirrhosis, acute pancreatitis, allograph rejection, allergic inflammation, contact dermatitis, delayed hypersensitivity, inflammatory bowel disease, septic shock, osteoporosis, osteoarthritis, neuronal inflammation, Osier-Weber syndrome, restenosis, fungal infection, parasitic infection, and viral infection.

10. A method for preparing the Crystalline Form II of Compound A anhydrate according to claim 2, which comprises stirring Compound A in an ester solvent with 2-10 carbon atoms, filtering, and vacuum drying the resultant solid to afford the crystal.
